Europäisches Patentamt

European Patent Office

Office européen des brevets

(11) Publication number:

**0 172 577**
**B1**

(12) **EUROPEAN PATENT SPECIFICATION**

(45) Date of publication of patent specification: **16.05.90**

(21) Application number: **85110560.1**

(22) Date of filing: **09.07.82**

(60) Publication number of the earlier application in accordance with Art. 76 EPC: **0 070 656**

(51) Int. Cl.⁵: **C 12 N 9/02,** A 61 K 37/50 // (C12N9/02, C12R1:43)

(54) **A superoxide dismutase, its production and pharmaceutical compositions containing it.**

(30) Priority: **22.07.81 US 285316**

(43) Date of publication of application: **26.02.86 Bulletin 86/09**

(45) Publication of the grant of the patent: **16.05.90 Bulletin 90/20**

(84) Designated Contracting States: **BE CH DE FR GB IT LI NL SE**

(56) References cited: **EP-A-0 045 222**

(73) Proprietor: **TAKEDA CHEMICAL INDUSTRIES, LTD.** 3-6, Doshomachi 2-chome Chuo-ku Osaka (JP)

(72) Inventor: **Miyata, Kouichi** 6-77, Seiwadainishi 1-chome Kawanishi Hyogo 666-01 (JP) Inventor: **Maejima, Kazutaka** 8-6, Daiwanishi 5-chome Kawanishi Hyogo 666-01 (JP) Inventor: **Tomoda, Katsumi** 17-2, Sakasedai 5-chome Takarazuka Hyogo 665 (JP)

(74) Representative: **Laredo, Jack Joseph et al Elkington and Fife Beacon House 113 Kingsway London, WC2B 6PP (GB)**

The file contains technical information submitted after the application was filed and not included in this specification

Courier Press, Leamington Spa, England.

**Description**

This invention relates to Mn-superoxide dismutase, its production and pharmaceutical composition.

The present invention undertook a diligent research for discovering a new superoxide dismutase and found that a microorganism belonging to the genus *Serratia* was able to produce such a new superoxide dismutase, as disclosed in the EP—A 81 303498.0 (publication number 0045222). This finding was followed by a further series of studies which have resulted in the present invention described in detail hereinafter.

This invention is directed to (1) a Mn-superoxide dismutase, which is produced by the microorganism *Serratia marcescens* ATCC 21074, (2) a method of producing a Mn-superoxide dismutase, in which superoxide dismutase is produced by the microorganism *Serratia marcescens* ATCC 21074, (3) an antiinflammatory agent which contains an effective amount of Mn-superoxide dismutase produced by the said method and (4) the use of said Mn-superoxide dismutase prepared by said method to produce a therapeutic preparation effective as an antiinflammatory agent.

In the present specification, the present superoxide dismutase produced by said microorganism *Serratia marcescens* ATCC 21074 belonging to the genus *Serratia* is sometimes referred to as "Serratia SOD" for the sake of brevity.

The said Serratia SOD is produced by cultivating a superoxide dismutase-producing microorganism belonging to the genus *Serratia,* namely *Serratia marcescens* ATCC 210974 in a culture medium to thereby cause the microorganism to elaborate and accumulate the superoxide dismutase in the culture medium and harvesting the superoxide dismutase from the resulting culture broth.

The above ATCC 21074 strain was deposited with the American Type Culture Collection, U.S.A. on May 15, 1967, and was listed in the American Type Culture Collection, Catalgoue of Strains I, Fourteenth Edition, 1980 and Fifteenth Edition 1982.

The morphological characteristics of *Serratia marcescens* E-15 ATCC 21074 are described in U.S. Patent No. 3,691,014 and Institute for Fermentation Research Communication No. 4, p. 1—11 (1969).

Microorganisms of the genus *Serratia,* in general, are liable to undergo change in characteristics and can be easily mutated by means of artificial treatments such as irradiation with X-rays, ultraviolet rays or radiation, mutagenic agents (nitrosoguanidine, ethyleneimine, etc.). Any of the resulting mutants can be employed for the purposes of this invention unless they have lost the ability to produce the superoxide dismutase.

The production of the contemplated superoxide dismutase is carried out by cultivating said microorganism in a culture medium and harvesting and purifying the resulting elaboration product, which is the desired product, from said culture medium.

The culture medium may be liquid or solid, provided it contains nutrient sources, which the particular strain of microorganism can utilize, although a liquid culture medium is desirable when mass production is contemplated.

Incorporated in such a medium are sources of nutrients which the microorganism can assimilate, digest or otherwise utilize, such as carbon sources, nitrogen sources, inorganic materials, trace nutrients and so on. The carbon sources may for example be starch, dextrin, glucose, sucrose, etc., and the nitrogen sources may for example by corn steep liquor, soybean meal, extracted soybean meal, yeast extract, denucleated yeast, casein, cotton seed meal, nitrogen-containing compounds such as ammonium chloride, ammonium sulfate, etc. The inorganic materials include, for example, sodium salts (e.g. sodium chloride), phosphates (e.g. sodium phosphate), calcium salts (e.g. calcium carbonate), potassium salts (e.g. potassium chloride, potassium phosphate), magnesium salts (e.g. magnesium sulfate), manganese salts (e.g. manganese chloride, manganese carbonate), iron salts (e.g. iron chloride), zinc salts (e.g. zinc carbonate) and so on. The trace nutrients may for example be vitamins (e.g. vitamin $B_1$, $B_2$), fatty acid esters [e.g. esters (e.g. methyl or ethyl esters) of oleic acid, lauric acid, caproic acid, etc.)], nucleic acids (e.g. ribonucleic acid, deoxyribonucleic acid), related compounds of nuelcic acids (e.g. inosinic acid, guanylic acid, adenylic acid) and so on. For deforming purposes, oils (e.g. soybean oil, corn oil, peanut oil), synthetic antifoams [e.g. Tween® 20, 60 and 80 (Kao-Atlas Co. Ltd., Japan), Actocol (Takeda Chemical Industries, Ltd., Japan)], etc. may also be incorporated.

The cultivation method may for example be stationary culture, shake culture or aerated stir (submerged aerobic) culture. Submerged aerobic culture, in particular, is especially useful for production-scale runs. While cultural conditions vary with the condition and composition of medium, strain of microorganism, cultural method, etc., cultivation is desirably conducted at about 20 to 40°C for about 10 to 100 hours, preferably, about 20 to 72 hours, the pH of the medium being about 4 to 9 and, preferably, about 6 to 8.

The desired enzyme is mostly accumulated intracellularly. This enzyme is harvested and purified by conventional purification procedures. It is advantageous to collect the cells from the culture broth by centrifugation, filtration or some other known separatory method, disrupt the cells by such a procedure as ultrasonication, grinding with glass beads, or a method involving the use of a surfactant or solvent, and extract the enzyme from the disrupted mass. In harvesting the enzyme, the same or other known separation and purification procedure in the field of art can be followed in any case to obtain a product of optional purity. Thus, the enzyme-containing disrupted mass is centrifuged or filtered to give a supernatant or filtrate. To this enzyme extract is added ammonium sulfate or acetone, and the resulting precipitate is collected by centrifugation, dissolved in a small quantity of water, desalted by dialysis and lyophilized, whereupon a crude enzyme powder is obtained. This crude enzyme powder can be purified by means employed conventionally for purification of enzymes. By way of illustration, gel filtration with Sephadex® (Pharmacia Fine Chemicals AB, Sweden), column chromatography on diethylaminoethyl-cellulose (Serva Fine Biochimica, West Germany) or a like procedure can be followed to isolate superoxide dismutase. In this manner, superoxide dismutase can be separated and isolated in a desired purity.

The enzymologial and chemical properties of the superoxide dismutase obtained in Example 1 below are as follows.

(a) Activity:
It dismutates a superoxide ion into a hydrogen peroxide molecule and an oxygen molecule.

(b) Substrate specificity:
It acts on superoxide ion.

(c) Optimum pH range:
The optimum pH range of this enzyme is about 7 to 8.

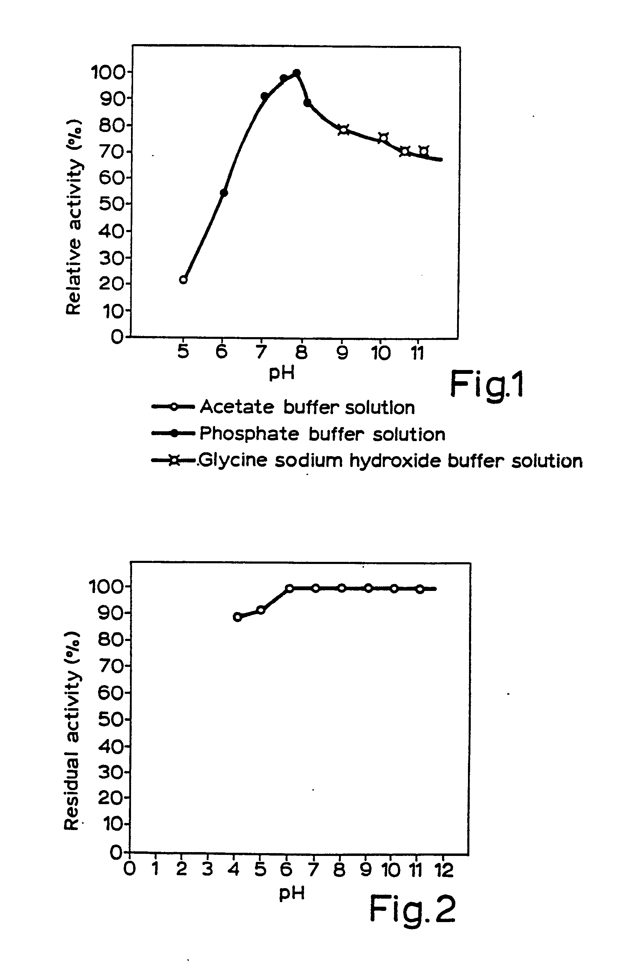

Fig. 1 shows a pH-activity curve of this enzyme.

(d) Stable pH range:
The enzyme is stable in the pH range of about 6 to 11.
Fig. 2 shows a pH-stability curve of this enzyme.

(e) Optimum functional temperature:
About 20 to 45°C

(f) Thermostability (conditions of inactivation):
The enzyme was treated at pH 7.8 and various temperatures for various time periods. The enzyme is stable at 37°C for 60 minutes but is inactivated about 50% when treated at 50 to 60°C for 60 minutes and almost 100% when treated at 80°C for 5 minutes.

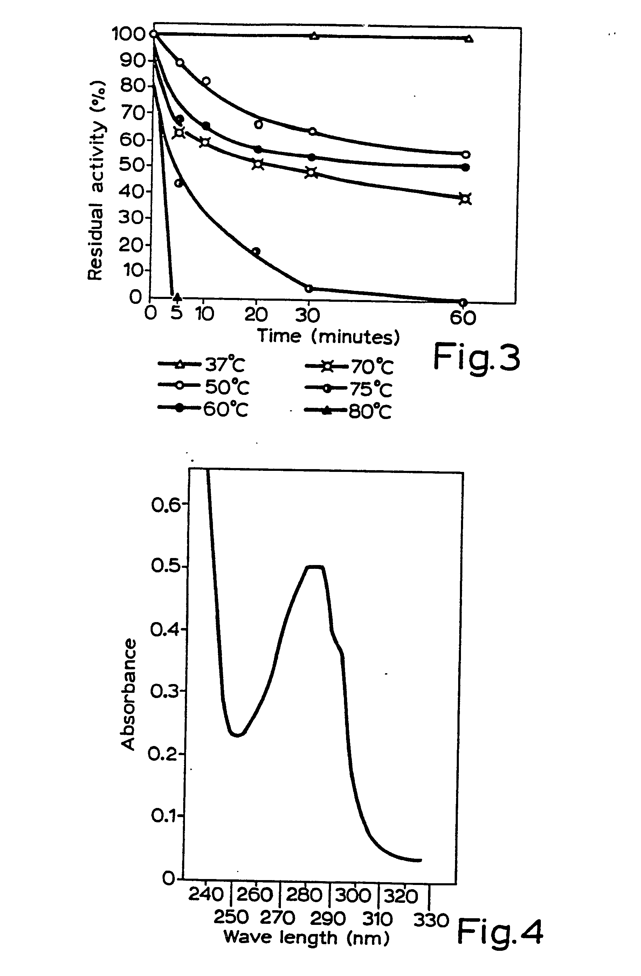

Fig. 3 shows the thermostability of this enzyme.

(g) Molecular weight:
As determined by the gel filtration method using a Sephadex® G-100 column [The Biochemical Journal 91, 222—233, 1964], this enzyme has a molecular weight of about $4.8 \times 10^4$. When the enzyme is first treated with 1% sodium laurylsulfate (SDS) and then subjected to polyacrylamide gel electrophoresis in the presence of the SDS [The Journal of Biological Chemistry 244, 5074—5080; 1969], the enzyme is found to have a molecular weight of about $2.4 \times 10^4$. These values indicate that this enzyme is a dimer consisting of subunits each having a molecular weight of about $2.4 \times 10^4$.

(h) Elemental analysis:
$C = 48.90 \pm 2.0\%$; $H = 7.05 \pm 2.0\%$; $N = 14.80 \pm 2.0\%$

(i) Absorptions in the ultraviolet and visible regions of the spectrum:

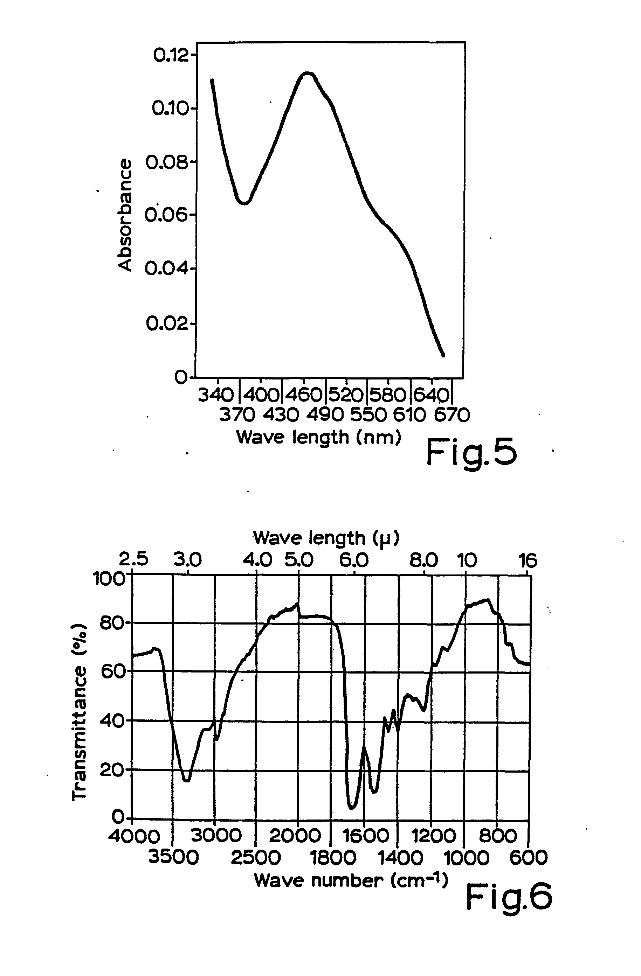

Fig. 4 shows an ultraviolet absorption spectrum of this enzyme and Fig. 5 shows a visible absorption spectrum of this enzyme.
In the UV region, this enzyme gives an absorption maximum at about 280 nm, an absorption minimum at about 253 nm and a shoulder at about 290 nm. The absorbance ratio (260 nm/280 nm) is 1.8. In the visible region, the enzyme shows an absorption maximum at about 470 to 475 nm, an absorption minimum at 375 to 380 nm and a shoulder at about 610 nm.

(j) Infrared absorption spectrum:
Fig. 6 shows an infrared absorption spectrum of this enzyme measured by the KBr method. Characteristic absorptions are 3.02(s), 3.38(m), 6.00(s), 6.60(s), 6.95(m), 7.25(m), 8.10(m), 8.50(w), 9.10(w).

(k) Molar extinction coefficient;
The molar extinction coefficient of the enzyme at 280 nm is about $1.08 \times 10^5 \text{ M}^{-1} \cdot \text{cm}^{-1}$.

(l) Amino acid analysis:
The enzyme is hydrolyzed with 6N-HCl at 110°C for a predetermined time period (24—72 hrs.) and analyzed for amino acids by means of an amino acid analyzer. The values given in the following table denote the number of amino acid residues per molecule of the enzyme (mol. wt. = $4.8 \times 10^4$).

| Amino acid | Number of residues | Amino acid | Number of residues | Amino acid | Number of residues |
|---|---|---|---|---|---|
| Lysine | 28.8 | Tryptophan | 14.4 | Serine | 20.2 |
| Histidine | 13.9 | Aspartic acid | 47.0 | Glutamic acid | 40.3 |
| Arginine | 10.6 | Threonine | 18.7 | Proline | 13.9 |
| Glycine | 23.5 | Valine | 22.6 | Leucine | 28.3 |
| Alanine | 48.4 | Methionine | 5.3 | Tyrosine | 18.2 |
| Cysteine | 0 | Isoleucine | 10.6 | Phenyl-alanine | 19.3 |

(m) Analysis for metal:

As determined by the atomic absorption method, this enzyme contains about 1.5 gram-atom of manganese. It is, therefore, clear that the enzyme is a Mn-superoxide dismutase.

(n) Isoelectric point is about pH 5.

The enzymatic potency assay procedure is as follows.

(1) The method described in the Journal of Biological Chemistry 244, 6049-6055 (1969) is employed. Thus, a sample cell (length of light pass 1 cm) is filled with 1.5 ml of 100 mM phosphate buffer (pH 7.8), 0.5 ml of 60µM cytochrome C, 0.5 ml of 0.6 mM ethylenediaminetetracetate sodium, 0.25 ml of 0.6 mM xanthine and 0.21 ml of the enzyme solution diluted with phosphate buffer (pH 7.8) to a measureable concentration, followed by addition of 0.04 ml of xanthine oxidase solution to make a total of 3.0 ml. This cell is set on a spectrophotometer and the reaction is intiated at 25°C so as to measure the initial velocity (v) or reduction of cytochrome C at 550 nm. As a blank, the initial velocity (V) of reduction of cytochrome C in the presence of distilled water in lieu of the enzyme solution is also measured. With the amount of the enzyme causing a 50% inhibition of the initial velocity of reduction of cytochrome C under the above reaction conditions being taken as unity, the enzymatic potency is calculated by means of the following equation.

$$U/mg = \frac{(V) - (v)}{(v) \times \text{amount of enzyme (mg)}/1.0 \text{ ml reaction mixture}}$$

(2) the active dye method described in Analytical Biochemistry 44, 276—287, 1971 can also be utilized. Thus, after polyacrylamide gel electrophoresis, the gel is immersed in 2.45 mM nitro blue tetrazolium for 20 minutes. Then, the gel is further immersed in a mixture of 28 mM tetraethylethylenediamine, 0.028 mM riboflavine and 36 mM potassium phosphate (pH 7.8) for 15 minutes. The gel is put in a test tube and exposed to the light rays of a 15-watt fluorescent lamp, whereupon the superoxide ion $O_2^-$ generated by photoreduction of riboflavine reduces the nitro blue tetrazolium so that the moiety other than that having superoxide dismutase activity forms a blue-formazan and is therefore dyed blue-purplish.

Proteins can be assayed by the method of Lowry [The Journal of Biological Chemistry 193, 265—275, 1951].

The superoxide dismutase according to this invention differs in physiochemical properties from the known superoxide dismutase. The known superoxide dismutase includes the one obtained from Escherichia coli B [The Journal of Bilogical Chemistry vol. 245, No. 22, pp. 6176—6181, 1970] and the one derived from Bacillus stearothermophilus [Journal of Molecular Biology, vol. 105, pp. 333—335, 1976]. The molecular weight of the superoxide dismutase derived from Escherichia coli is 39,500 and that of the superoxide dismustase from Bacillus stearothermophilus is 40,000. Since these weight figures are significantly different from the molecular weight of the enzyme of this invention which is about $4.8 \times 10^4$, the superoxide dismutase according to this invention is considered to be a novel enzyme.

Serratia SOD has an antiinflammatory activity. Thus, for instance, when tested by the commonly known carrageenan-induced edema method [The Journal of Pharmacology and Experimental Therapeutics, 150, 328 (1965)], Serratia SOD (a sample having an activity of 3,000 U/mg) shows an overt inhibition of edema formation in the rat hind leg at a dose of 1.0 mg/kg or more under intravenous administration.

Effect on nystatin edema:

A 1% (w/v) nystatin solution in physiological saline was injected subcutaneously into the paw of JCL-ICR mice at a dose of 0.025 ml. At the same time, Serratia SOD (about 11,000 to 12,000 U/mg protein) was administered to the same site by subcutaneous injection. This experiment was conducted according to the method described in Pharmacology, vol. 5, pp. 215—224 (1971). After six hours from the nystatin administration, the edema volume was measured. The results are set forth in the following table.

## Table 1

| Treatment | Dose U/paw | No. of mice | Edema volume (mg) Mean ± S.E. | Inhibition (%) |
|---|---|---|---|---|
| Control | - | 10 | 120.45 ± 8.87 | - |
| Serratia SOD | 50 | 10 | 92.35 ± 4.38 | 23.3 |
| Serratia SOD | 500 | 10 | 83.71 ± 3.28 | 30.5 |

S.E. = standard error

As is clear from the above results, Serratia SOD inhibits nystatin edema.

Serratia SOD is sparingly toxic and no death is encountered even when it is intraperitoneally administered to rats at a dose of 60 mg/kg. Even when 625 mg/kg of Serratia SOD was intrapertioneally administered to mice, no death was encountered. Furthermore, when Serratia SOD was intraveneously administered to mice at a dose of 2,000 mg/kg, no death was observed.

Serratia SOD has an antiinflammatory activity as mentioned above and accordingly is of use as an antiinflammatory agent.

The antiinflammatory agent contains an effective amount of Serratia SOD as an active ingredient in association with a pharmaceutically acceptable carrier or exceipient therefor.

For the treatment of various acute or subacute inflammatory diseases or edema (e.g. arthritis, bronchitis, edema due to bruise, burn) in mammalian animals (e.g. mouse, rat, rabbit, cat, dog, monkey, man), Serratia SOD can be administered orally or parenterally as formulated in a conventional manner into tablets, capsules, injections, ointments, or other dosage forms at a daily dose of about 1 to 5 mg (as enzyme preparation) per kg.

Examples of the carrier for use in preparing the above phrmaceutical preparations are albumin, globulin, dextran, Ficoll® Type 70 (polymer of sucrose, molecular weight about 70,000), Ficoll® Type 400 (polymer of sucrose, molecular weight about 400,000) (Sigma Chemical Company, U.S.A.), lactose, dextran, and starch. The production of pharmaceutical preparations may be carried out by employing a *per se* known method.

The following Examples are given to illustrate this invention in further detail without limiting its scope. Unless otherwise specified, percents (%) are weight/volume percents (w/v %).

## Example 1

(a) Harvesting the cells

A 2000 ml Sakaguchi flask was filled with 500 ml of a liquid medium containing 1.0% of solvent-extracted soybean meal, 1.5% of casein, 0.6% of diammonium phosphate, 0.1% of sodium chloride, 0.05% of potassium chloride, 0.02% of calcium chloride, 0.02% of magnesium sulfate, 1.0% of calcium carbonate, 0.0021% of zinc carbonate, 0.6% of soybean oil and 0.1% of Actocol (adjusted to pH 7.0), and, after sterilization, inoculated with *Serratia marcescens* ATCC 21074. The flask was incubated at 28°C on a rotary shaker revolving at 230 r.p.m. for 24 hours to give a seed culture. A 200-liter tank was charged with 100 l of a medium of the same composition as above and under sparging at the rate of 100 l/min., submerged aerobic culture was conducted at 28°C. The resulting fermentation broth was centrifuged to separate the cells which were lyophilized at −20°C.

(b) Preparing a crude enzyme product

A 5 kg portion of the wetted cell was reconstituted at 30°C and after addition of 10 l of 20 mM phosphate buffer (pH 7.8), the cells were disrupted in a Dynomill apparatus and centrifuged in a Sharples type centrifuge to give a clear supernatant. From this supernatant, the precipitate obtained with 30—70%

EP 0 172 577 B1

(W/V) of ammonium sulfate was collected by centrifugation. This sediment was dissolved in 20 mM phosphate buffer containing a small amount of 0.1 M KCl (pH 7.0) and, in a cellulose tube, dialyzed against the same type of buffer for 3 days. The internal fluid, i.e. 530 ml of dialysate, was concentrated to 40 ml by ultrafiltration through a Diaflow-UM-5 membrane. The concentrate was loaded onto a column (4.0 × 66.0 cm) of Sephadex® G-150 previously equilibrated with the same buffer solution and eluted with the same buffer to collect 550 ml of an enzymatically active fraction. To this fraction was added ammonium sulfate to a concentration of 70% (W/V) and the resultant precipitate was collected by centrifugation and dissolved in a small amount of water. In a cellulose tube, this solution was dialyzed against water for 3 days and the internal fluid was lyophilized to give 2070 mg of a crude enzyme product. The specific activity of this product was 3600 U/mg. protein.

(c) Preparing a pure enzyme product
   (i) Column chromatography on diethylaminoethyl-cellulose
   In a small amount of 20 mM tris-HCl buffer (pH 9.0) was dissolved 2070 mg of the crude enzyme obtained in (b), and in a cellulose tube, the solution was dialyzed against the same buffer for 3 days. The internal fluid, i.e. 134 ml of dialysate, was put on a column (3.8 × 55.0 cm) of diethylaminoethyl-cellulose previously equilibrated with the same buffer and the enzyme was eluted by increasing the concentration of sodium chloride in the same buffer linearly from 0 to 250 mM to collect 54 ml of an enzymatically active eluate
   (ii) Gel filtratration on Sephadex® G-100
   The enzyme solution obtained in (i) (54 ml) was put in a cellulose tube and dialyzed against 20 mM phosphate buffer containing 0.1 M of KCl. The internal fluid (73 ml) was concentrated to 2.0 ml by ultrafiltration through a Dyaflow UM-5 membrane. This concentrate was put on a column (2.6 × 74.0 cm) of Sephadex® G-100 equilibrated with the same buffer as above and the enzyme was eluted with the same buffer to collect an enzymatically active eluate which was red-purple in color. The fractions thus obtained were constant in specific activity, and polyacrylamide gel electrophoresis shows that it was a single component product. The specific activity of this Serratia SOD (2.07 mg) was 12,000 U/mg-protein.

Example 2
Enteric capsules are prepared by a conventional method employing the following ingredients:

| | |
|---|---|
| Serratia SOD prepared in Example 1 (3,600 U/mg) | 10 mg |
| Lactose | 46 mg |
| Corn Starch | 16 mg |
| Crystalline cellulose | 12 mg |
| Cellulose glycollic acid | 5 mg |
| Sorbitol | 10 mg |
| | 99 mg (per capsule) |

The daily dose of the above capsules for human adults is genrally 2 capsules after each meal (3 times a day).

Example 3
Enteric tablets are produced by a conventional method employing the following ingredients:

| | |
|---|---|
| Serratia SOD obtained in Example 1 (3,600 U/mg) | 10 mg |
| Lactose | 79 mg |
| Corn Starch | 45.5 mg |
| Magensium stearate | 0.5 mg |
| | 135 mg (per tablet) |

6

The daily dose of the above tablets for human adults is generally 2 tablets after each meal (3 times a day).

**Claims**

1. A Mn-superoxide dismutase produced by *Serratia marcescens* ATCC 21074, which has the following properties:

(a) Activity: to dismutase a superoxide ion into a hydrogen peroxide molecule and an oxygen molecule;

(b) Substrate specificity: to act on superoxide ion;

(c) Optimum pH range: about 7 to 8;

(d) Stable pH range: about 6 to 11;

(e) Optimum functional temperature range: about 20 to 45°C;

(f) Thermostability: Stable at 37°C for 60 minutes; Inactivated about 50% at 50—60°C for 60 minutes; Inactivated about 100% at 80°C in 5 minutes;

(g) Molecular weight: about $4.8 \times 10^4$ (by gel filtration method);

(h) Elemental analysis: C 48.90 $\pm$ 2.0%;

H 7.05 $\pm$ 2.0%;

N 14.80 $\pm$ 2.0%;

(i) Absorptions in the visible region of the spectrum: 470—475 nm (max.), 3756—380 nm (min.), about 610 nm (shoulder);

(j) molar extinction coefficient: about $1.08 \times 10^5 M^{-1} \cdot cm^{-1}$ at 280 nm;

(k) Amino Acid analysis

| Amino acid | Number of residues |
|---|---|
| Glycine | 23.5 |
| Leucine | 28.3 |

(The values denote the number of amino acid residues per molecule of the enzyme).

2. Method of producing a Mn-superoxide dismutase according to Claim 1, characterised by cultivating *Serratia marcescens* ATCC 21074 in a culture medium to thereby cause the microorganism to elaborate and accumulate said Mn-superoxide dismutase and harvesting said Mn-superoxide dismutase from the resulting culture broth.

3. An antiinflammatory agent which contains an effective amount of a Mn-superoxide dismutase according to Claim 1.

4. The use of Mn-superoxide dismutase according to Claim 1 to produce a therapeutic preparation effective as an antiinflammatory agent.

**Patentansprüche**

1. Mn-superoxid-Dismutase, hergestellt von Serratia marcescens ATCC 21074 mit den folgenden Eigenschaften:

(a) Aktivtät: ein Superoxid-Ion in ein Hydrogenperoxid-Molekül und ein Sauerstoff-Molekül zu dismutieren;

(b) Substrat-Spezifität: auf ein Superoxid-Ion zu wirken;

(c) optimaler pH-Bereich: etwa 7 bis 8;

(d) stabiler pH-Bereich: etwa 6 bis 11;

(e) optimaler funktionaler Temperatur-Bereich: etwa 20°C bis 45°C;

(f) Thermostabiltät 60 min stabil bei 37°C; zu 50% inaktiviert nach 60 min bei 50°C bis 60°C; zu 100% nach 5 min bei 80°C;

(g) Molekulargewicht: etwa $4,8 \times 10^4$ (mittels der Methode der Gelfiltration);

(h) Elementaranalyse: C 48,90 $\pm$ 2,0%,

H 7,05 $\pm$ 2,0%,

N 14,80 $\pm$ 2,0%;

(i) Absorptionen im sichtbaren Bereich des Spectrums: 470—475 nm (max.), 375—380 nm (min.), etwa 610 nm (Schulter);

(j) molarer Extinktionskoeffizient: etwa $1,08 \times 10^5 mol^{-1} cm^{-1}$ bei 280 nm;

(k) Aminosäure-Analyse:

| Aminosäure | Zahl der Reste |
|---|---|
| Glycin | 23,5 |
| Leucin | 28,3 |

(Die Werte bezeichnen die Anzahl der Aminosäure-Reste pro Molekül des Enzyms.

2. Verfahren zur Herstellung einer Mn-superoxid-Dismutase nach Anspruch 1, dadurch gekennzeichnet, daß Serratia marcescens ATCC 21074 in einem Kulturmedium kultiviert wird, um dadurch den Mikroorganismus zur Bildung und Anreicherung der Mn-superoxid-Dismutase zu veranlassen, und die Mn-superoxid-Dismutase aus der resultierenden Kulturbrühe geerntet wird.

3. Antiinflammatorisches Mittel, enthaltend ein wirksame Menge einer Mn-superoxid-Dismutase nach Anspruch 1.

4. Verwendung einer Mn-superoxid-Dismutase nach Anspruch 1 zur Erzeugung eines therapeutischen Präparats, das als antiinflammatorisches Mittel wirksam ist.

**Revendications**

1. Peroxyde dismutase Mn produite par *Serratia marcescens* ATCC 21074, qui présente les propriétés suivantes:

(a) Activité: la dismutation d'un ion peroxyde en une molécule de peroxyde d'hydrogène et une molécule d'oxygène;

(b) Spécificité du substrat: l'action sur l'ion peroxyde;

(c) Domaine optimum de pH: environ 7 à 8;

(d) Domaine stable de pH: environ 6 à 11;

(e) Domaine optimum de température fonctionnelle: environ 20 à 45°C;

(f) Stabilité thermique: stable à 37°C pendant 60 minutes; inactivée à environ 50% à 50—60°C en 60 minutes; inactivée à environ 100% à 80°C en 5 minutes;

(g) Poids moléculaire: environ $4,8 \times 10^4$ (par la méthode de filtration sur gel);

(h) Analyse élémentaire: C $48,90 \pm 2,0\%$;

H $7,05 \pm 2,0\%$;

N $14,80 \pm 2,0\%$;

(i) Absorptions dans la région visible du spectre: 470—475 nm (max.), 375—380 nm (min.), environ 610 nm (épaule);

(j) Coefficient d'extinction moléculaire: environ $1,08 \times 10^5$ $M^{-1} \cdot cm^{-1}$ à 280 nm;

(k) Analyse des acides aminés:

| Acide aminé | Nombre des restes |
| --- | --- |
| glycine | 23,5 |
| leucine | 28,3 |

(Les valeurs indiquent le nombre de restes d'acide aminé par molécule de l'enzyme).

2. Procédé de préparation de peroxyde dismutase Mn selon la revendication 1, caractérisé en ce que l'on cultive *Serratia marcescens* ATCC 21740 dans un milieu de culture de façon à faire produire et accumuler par le microorganisme ladite peroxyde dismutase Mn, et l'on récolte ladite peroxyde dismutase Mn à partir du bouillon de culture résultant.

3. Agent anti-inflammatoire qui contient une quantité efficace de peroxyde dismutase Mn selon la revendication 1.

4. Utilisation de la peroxyde dismutase Mn selon la revendication 1 pour la préparation d'une composition thérapeutique efficace comme agent anti-inflammatoire.

EP 0 172 577 B1

Fig.1

—o— Acetate buffer solution
—●— Phosphate buffer solution
—☆— Glycine sodium hydroxide buffer solution

Fig.2

1

Fig.3

Fig.4

Fig.5

Fig.6